# EUROPEAN PATENT SPECIFICATION

(11) **EP 2 150 529 B1**
(45) Date of publication and mention of the grant of the patent: **27.03.2013**
(21) Application number: 08758729.1
(22) Date of filing: 23.05.2008
(51) Int. Cl.: C07D 201/04

(54) **METHOD OF MAKING A LACTAM IN AN IONIC LIQUID**
VERFAHREN ZUR HERSTELLUNG EINES LACTAMS IN EINER IONISCHEN FLÜSSIGKEIT
PROCÉDÉ DE PRÉPARATION D'UN LACTAME DANS UN LIQUIDE IONIQUE

(30) Priority: 25.05.2007 EP 07010470
(43) Date of publication of application: 10.02.2010
(73) Proprietor: DSM IP Assets B.V., 6411 TE Heerlen (NL)
(72) Inventor: BONRATH, Werner, 79115 Freiburg (DE); LEVEQUE, Jean-Marc, F-73470 Novalaise (FR); RAGAINI, Vittorio, I-20149 Milano (IT); TURGIS, Raphael, F-91940 Gometz le Chatel (FR)
(74) Representative: Kurt, Manfred
(86) International application number: PCT/EP2008/004136
(87) International publication number: WO 2008/145312

(56) References cited:
- CN-A- 1 852 898
- GUI J ET AL: "A novel task-specific ionic liquid for Beckmann rearrangement: a simple and effective way for product separation" TETRAHEDRON LETTERS, ELSEVIER, AMSTERDAM, vol. 45, no. 12, 15 March 2004 (2004-03-15), pages 2681-2683, XP004492808 ISSN: 0040-4039
- DATABASE WPI Week 200532 Thomson Scientific, London, GB; AN 2005-314913 XP002494798 & WO 2005/028446 A (null) 31 March 2005 (2005-03-31)
- DATABASE WPI Week 200701 Thomson Scientific, London, GB; AN 2007-000291 XP002494799 & CN 1 781 908 A (CHINESE ACAD SCI LANZHOU CHEM PHYS INST) 7 June 2006 (2006-06-07)
- JAE KYUN LEE ET AL.: SYNTHETIC COMMUNICATIONS, vol. 33, no. 13, 2003, pages 2301-2307, XP009105452
- DATABASE WPI Week 200781 Thomson Scientific, London, GB; AN 2007-874300 XP002494800 & CN 1 978 427 A (CHINESE ACAD SCI LANZHOU CHEM & PHYS) 13 June 2007 (2007-06-13)

## Description

The invention relates to a method of contacting a cyclic oxime with a Brønsted acidic ionic liquid having a sulfur atom in the anionic portion and/or the cationic portion further comprising mechanical stirring, microwave irradiation and/or ultrasonic treatment.

Lactams are well known precursors for nylon materials, such as ε-caprolactam as a precursor of nylon 6, which worldwide production is close to 5.5 tonnes/year. ε-caprolactam is industrially produced via the Beckmann rearrangement that is not only one of the most well known reactions in synthetic organic chemistry but still remains one of the most studied chemical transformation to access to amides from oximes. However, the industrial route to ε-caprolactam requires concentrated sulfuric acid and high temperature and involves needs for reducing environmental burden and ensuring safety.

Of course, alternative methods have been explored in either vapour phase or liquid phase systems. In the past, various types of catalysts have been designed to enhance the reactivity, among them boro-hydroxyapatites, metals ilerites, supported oxides and zeolites including MCM-41, MCM-22, SAPO-11 and MgCoAlPO-36. Despite high conversion and selectivity in ε-caprolactam obtained, the vapour phase rearrangement requires high temperatures (250 - 300°C) for catalyst transformation leading to a rapid decrease of the catalytic activity and high energetic comsumption, which is also an environmental and economic problem. But, good conversion and selectivity can be obtained with lesser drastic conditions of temperature using sulfamic acid, chloral, anhydrous oxalic acid, solid metaboric acid, antimony salts or chlorosulfonic acid (such as Tetrahedron lett. 2005, 46, 671) in toluene. Nevertheless, the neutralisation of such acids is unavoidable and the formation of mineral salts hampers the full recovery of ε-caprolactam with a 10-15% lost of product. Therefore, both vapour and liquid phases are on road to green chemistry but are still far from. This latter recommends, through twelve major principles, to develop an organic synthetic chemistry respecting as much as possible the environment by the use of less hazardous and toxic chemicals together with a total control (time, temperature, pressure, energy, etc.) of the expected chemical transformation. Also Green Chemistry rules recommend the use of safer and/or recyclable solvents than common organic molecular solvents. Alternatives have been developed in recent years and the use of ionic liquids or supercritical fluids appear both as reasonable substitutes for the former.

The Beckmann rearrangement has already been studied in supercritical water. Good selectivity is obtained but a very low conversion of cyclohexanone oxime (30%) is observed making this method unusable for industrial applications. Moreover, ε-caprolactam is poorly soluble in supercritical CO₂ (0.07 in molar fraction).

Room temperature ionic liquids have received recognition of benign media for the environment and are promising solvents and / or catalysts for the organic synthesis. So far their use in the synthesis of ε-caprolactam was described in very few studies. Indeed, some couples RTIL / catalyst such as [BPy]BF₄ with PCl₅, [BMIM]PF₆ or [BMIM]BF₄ with P₂O₅ or the Eaton's reagent, [BMIM]PF₆ with metaboric acid give good average conversion and selectivity. Unfortunately, extraction problems hamper the overall process by avoiding the recycling of the RTIL. The second application was their use as dual solvent and catalyst. In this way, a third generation of ionic liquids, called TSILs (Task Specific Ionic Liquid) was especially designed by incorporating a sulfonyl chloride function. (Scheme 1).

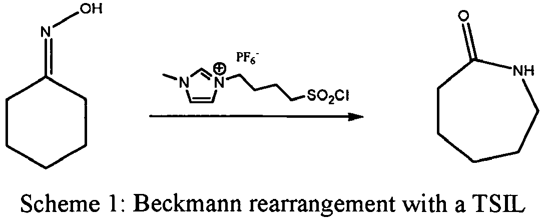

Very good results were then obtained in reasonable time and temperature conditions. Nevertheless conversion was dramatically decreased after the first recycling due to the partial hydrolysis of the sulfonyl chloride group into a sulfonic acid group.

Very recently, Guo et al. (Green Chem. 2006, 8, 296) studied the ε-caprolactam synthesis using a new TSIL, consisting in a ε-caprolactam based cation itself (Scheme 2).

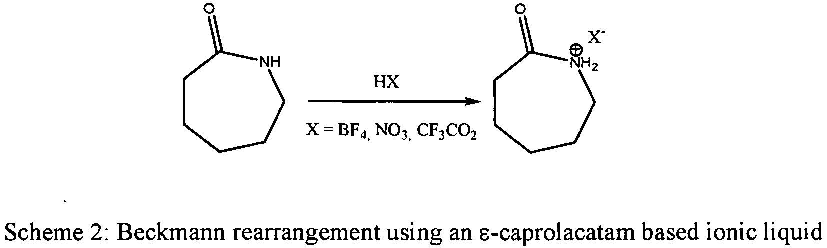

As before, good results were obtained in reasonable time and temperature conditions. Nevertheless no recycling is mentioned whereas toxic organic solvents, such as benzene and CCl₄ are used to extract ε-caprolactam based cation.

Gui et al (Tetrahedron Letters 2004, Vol. 45. No.12, p.2681 - 2683) discloses a novel task-specific ionic liquid for Beckmann rearrangement, using TISC as ionic liquid.

WO2005/028446 is disclosing ionic liquids with a SO₂Cl group and reaction processes for the use of them.

Surprisingly, it was found that the reaction can be improved and recyclability guaranteed by using a Beckmann rearrangement, wherein a cyclic oxime reacts with a Brønsted acidic ionic liquid of formula 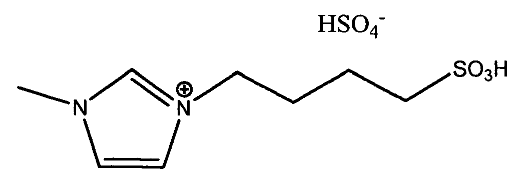 further comprising:
a) mechanical stirring of more than 3 hours at more than 70°C wherein the ratio of cyclic oxime to bronsted acidic ionic liquid is between 1:1 to 1:10 and/or
b) microwave irradiation of more than 30 s, preferably more than 1 min, more preferably between 1 min and 20 min at temperatures of more than 120°C, preferably between 120°C and 250°C and/or
c) ultrasonic treatment of more than 5 s at more than 70 °C at a frequency of between 18 and 800 kHz, preferably 20 to 300 kHz, more preferably 20 to 50 kHz.

Brønsted acidic ionic liquids can be synthesised (Scheme 3) according to the literature.

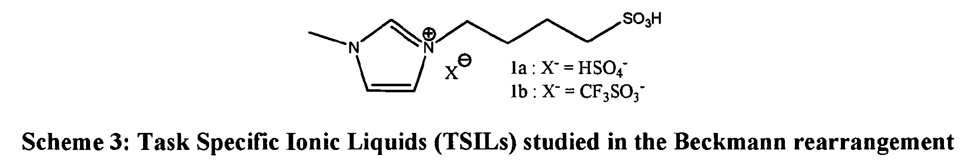

In order to better understand the behaviour and the catalytic activity of these ionic liquids, experiments on temperature, time, ratio of ionic liquids over cyclohexanone oxime, and anion effects were conducted.

The effect of temperature was studied and the results are summarized in table 1.

**Tableau 1: Temperature effect on the Beckmann rearrangement in ultrasound-)))- , microwave-MW- and mechanical stirring-(MS)- conditions. TSIL: [HO₃SBMIM]HSO₄ / oxime = 1 / 1**

| Entry | Activation method | T (°C) | Time | ε-caprolactam | Conversio n of oxime |
|---|---|---|---|---|---|
| 1 | MS | 80 | 6h | 46% | 62% |
| 2 | MS | 100 | 6h | 62% | >99 % |
| 3 | )))(20 kHz) | 70 → 190 | 10 s | 68 % | 77 % |
| 4 | MW | 45 → 210 | 4 min | 95 % | 98 % |
| 5 | MW | 45 → 120 | 10 min | 0% | / |

| | | | | | |
|---|---|---|---|---|---|
| ***Secondary products including: cyclohexenone oxime ; 2-(1-cyclohexenyl) cyclohexanone ; 2-cyclohexylidene cyclohexanone** | | | | | |

The entries 1/2 of this first table show a great increase of the conversion under mechanical stirring at different temperatures which confirms a thermal demand of the Beckmann rearrangement. This thermal demand is also confirmed in entries 4/5 under microwaves irradiation since the reaction is completed in 4 minutes at 210°C and does not occur at 120°C in 10 minutes.

The ultrasonic mediated reaction (entry 3) reveals a very promising result, leading to 77% of ε-caprolactam in 10 seconds. Polymerisation of ε-caprolactam can already be observed under microwave irradiation when the power level is very high (entry 4) and under mechanical stirring with a temperature over 100°C.

These first results show that the temperature is not only a key parameter, but also that a fine monitoring of the reaction is necessary, if one wants to avoid the polymerisation phenomenon of ε-caprolactam.

Next, we studied the effect of temperature with various bronsted acidic ionic liquids.

The effect of the anion was studied with an ionic liquid based on the same cation in which the anion hydrogen sulphate is substituted by the triflate anion. Results are summarized in table 2:

**Table 2: Anion effect on the Beckmann rearrangement in ultrasound, microwave and mechanical stirring conditions. cation: [HO₃SBMIM]⁺/ oxime = 1 / 1**

| Anion | Entry | Activation method | T (°C) | Time | ε-caprolactam | Conversio n of oxime |
|---|---|---|---|---|---|---|
| CF₃S O₃⁻ | 1 | MS | 80 | 6h | 2% | 3% |
| | 2 | MS | 100 | 6h | 59 % | 70% |
| | 3 | MW | 45 → 220 | 4 min | 76% | 78 % |
| | 4 | MW | 45 → 120 | 10 min | 0% | / |
| HSO₄⁻ | 5 | MS | 80 | 6h | 46% | 62% |
| | 6 | MS | 100 | 6h | 62% | >99 % |
| | 7 | MW | 45 → 210 | 4 min | 95 % | 98 % |
| | 8 | MW | 45 → 120 | 10 min | 0% | / |

| | | | | | | |
|---|---|---|---|---|---|---|
| ***Secondary products including: cyclohexenone oxime ; 2-(1-cyclohexenyl) cyclohexanone ; 2-cyclohexylidene cyclohexanone** | | | | | | |

Results are better with the hydrogen sulphate anion than triflate anion whatever the experimental conditions are.

We then synthesized a non task specific ionic liquid such as the butylmethylimidazolium hydrogen sulphate: [BMIM]HSO₄ without the sulfonic acid function and results are presented in table 3:

**Table 3: Study of the influence of the acidic proton of the hydrogen sulphate anion. Conditions: Mechanical stirring, 6 hours, ratio IL / oxiome = 1/1.**

| Ionic Liquid | Temperature | ε-caprolactam | Conversion of oxime |
|---|---|---|---|
| [BMIM]HSO₄ | 80 °C | 0% | 0% |
| | 120 °C | 0% | 0% |
| [H0₃SBMIM]HSO₄ | 100 °C | 62% | >99% |

In [BMIM]HSO₄ no reaction occurs whatever temperature is, whereas 59% of ε-caprolactam are synthesized at 100°C using [BMIMSO₃H]HSO₄, showing the great role of the sulfonic group on the alkyl chain in the Beckmann rearrangement catalysis.

Further the influence of ionic liquid/ cyclohexanone oxime ratio was studied and the results are summarized in table 4.

**Table 4: effect of the ratio TSIL / oxime. Cation : [HO₃SBMIM]⁺, mechanical stirring.**

| Anion | Entry | T (°C) | Time | TSIL/ Oxime | ε-caprolactam | Conversion of oxime |
|---|---|---|---|---|---|---|
| HSO₄⁻ | 1 | 100 | 6h | 1/1 | 46% | 63 % |
| | 2 | 80 | 4h | 1/1 | 11 % | 25 % |
| | 3 | 80 | 4h | 3 / 1 | 63 % | 66% |
| | 4 | 80 | 4h | 5/1 | >99% (96)* | >99% |
| CF₃SO₃⁻ | 5 | 100 | 6h | 1/1 | 59 % | 70% |
| | 6 | 80 | 6h | 1/1 | 2% | 3% |
| | 7 | 80 | 4h | 3/1 | 47 % | 50 % |
| | 8 | 80 | 4h | 5/1 | 73 % | 75 % |

| | | | | | | |
|---|---|---|---|---|---|---|
| *** Isolated yield** | | | | | | |

Results in table 4 show that TSIL/oxime ratio is a crucial parameter of the system. More important is the amount of TSIL versus cyclohexanone oxime, more easily and faster occurs the rearrangement and better is the selectivity (entries 2/4 and 6/8 - table 4). This effect is seen whatever the anion. By gradually increasing the ratio, conversion and selectivity increase also both up to a quantitative conversion of cyclohexanone oxime into ε-caprolactam without polymerisation. (entry 4)

The good results obtained with an excess of ionic liquid is not due to the displacement of the reaction equilibrium since no kinetic product is detected, such as 2-cyclohexylidene cyclohexanone, but is due, as we said before, to an increase of the internal pressure, thus decreasing the thermodynamic barrier. This might explain why only the thermodynamic product, ε-caprolactam, is formed. (entries 3/4 and 7/8)

Finally, the difference of conversion yields between triflate and hydrogen sulphate anions based ILs is again observed in favour of the former. (entries 4/8 - table 4) The nature of the used ionic liquid is so, as thought, a very important parameter.

The effect of the cation in this reaction was studied and the results are presented in table 5.

Three kind of cation were tested: methylimidazolium [HO₃SBMIM]⁺, 1,2 dimethylimidazolium [HO₃SBDMIM]⁺ and pyridinium [HO₃SBPy]⁺ cations.

**Table 5: Effect of the cation. Conditions: anion : HSO₄⁻, mechanical stirring, 4 hours, 80°C, ratio TSIL / oxime : 5/1**

| Entry | Cations | ε-caprolactam | Conversion of oxime |
|---|---|---|---|
| 1 | BMIM | >99% | >99% |
| 2 | 1,2 BDMIM | 82% | 83 % |
| 3 | BPy | >99% | > 99% |

These results show an equivalent activity between the methylimidazolium and pyridinium cations

A difference is obtained when the butyl-1,2-dimethylimidazolium cation is used, that can be ascribable to sterical or physico-chemical reasons. Indeed, the methyl group fixed on C2 position of the butyl-1,2-dimethylimidazolium cation may obstruct the approach of the cyclohexanone oxime leading to a lower conversion than with the other cations.

But, it is also known that the hydrogen in the C2 position in the methyl imidazolium cation possesses some acidic properties, maybe interfering in the rearrangement mechanism. This may explain why the conversion yield decreases when this later is replaced by a methyl group.

The recyclability was tested, which was not observed in the prior art.

After reaction, the mixture is dissolved in water and ε-caprolactam is extracted by diethyl ether. The water is removed under vacuum (2 hours 100°C) and cyclohexanone is added to the dried ionic liquid. Results are presented in table 6.

**Table 6: Recycle. Conditions: TSIL: [HO₃SBMIM]HSO₄, mechanical stirring, 4 hours, 80°C, ratio TSIL / oxime : 5/1**

| Cycle | ε-caprolactam | Conversion of oxime |
|---|---|---|
| 1 | >99% | >99% |
| 2 | >99% | >99% |
| 3 | 50% | 54 % |

The recycling of the ionic medium is possible as shown here above. Nevertheless, the entry 3 showss that the ionic liquid must be carefully dried prior to reuse as the oxime is easily hydrolysed into cyclohexanone by remaining water avoiding its conversion into ε-caprolactam.

### Examples:

The following examples illustrate the invention:
Zwitterions synthesis:

1-H-Methylimidazole (8.21g, 0.1 mol) or 1,2 dimethylimidazole (9.61g, 0.1 mol) or pyridine (7.91g, 0,1 mol) and 1,4-butane sultone (13.62g, 0.1 mol) were charged into a 50 mL round bottom flask and stirred at 40°C for 10 hours. The white solid zwitterion was washed repeatedly with toluene (3x10 mL), cyclohexane (3x10 mL) and ether (5x10 mL) and dried under vacuum. The zwitterions were formed quantitatively.

Task specific ionic liquids synthesis:
[HO₃S-BMIM]HSO₄:
   A stoichiometric amount of sulphuric acid (95%) was carefully added dropwise over the zwitterions without solvent. Then the mixture was stirred 6 hours at 80°C under argon atmosphere. The viscous colourless ionic liquid obtained was washed repeatedly with toluene (3x10 mL), cyclohexane (3x10 mL) and ether (5x10 mL) and dried under vacuum for 3 hours at 80°C. Quantitative yield is obtained.
NMR (BRUKER 300 MHz AMX, CDCl₃):
   **¹H: 1,67 ppm (m, 2H); 1,88 ppm (m, 2H); 3,00 ppm (t, 2H); 3,72 ppm (s, 3H); 4,06 ppm (t, 2H); 7,26 ppm (d, 1H); 7,32 ppm (d, 1H); 8,48 ppm (s, 1H); 11,42 ppm (s, 1H)**
[HO₃ S-BDMIM]HSO₄:
   Same protocol as [HO₃S-BMIM]HSO₄.
   NMR (BRUKER 300 MHz AMX, CDCl₃):
      **¹H: 1,80 ppm (m, 2H) ; 2,00 ppm (m, 2H) ; 2,90 ppm (t, 2H) ; 3,85 ppm (s, 3H) ; 4,2 ppm (t, 2H) ; 5,85 ppm (s, 3H) ; 7,50 ppm (d, 1H) ; 7,55 ppm (d, 1H); 11,52 ppm (s, 1H)**
[HO₃S-BPy]HSO₄:
   Same protocol as [HO₃S-BMIM]HSO₄.
   NMR (BRUKER 300 MHz AMX, CDCl₃):
      **¹H :1,80 ppm (2H) ; 2,10 ppm (2H) ; 3,10 ppm (2H) ; 4,50 ppm (2H) ; 7,90 ppm (2H) ; 8,40 ppm (1H); 8,70 ppm (2H) ; 11,80 ppm (1H)**
   [HO₃ S-BMIM]OTf:
A stoichiometric amount of triflic acid (99 %) was carefully added dropwise over the zwitterions without solvent. Then the mixture was stirred 2 hours at 40°C under argon atmosphere. The slighty purple viscous ionic liquid obtained was washed repeatedly with toluene (3x10 mL), cyclohexane (3x10 mL) and ether (5x10 mL) and dried under vacuum for 3 hours at 80°C. This ionic liquid crystallizes with a melting point < 60°C. Quantitative yield is obtained.
   NMR (BRUKER 300 MHz AMX, CDCl₃):
   **¹H : 1,80 ppm (m, 2H); 2,00 ppm (m, 2H); 3,10 ppm (t, 2H); 3,80 ppm (s, 3H); 4,20 ppm (t, 2H); 7,30 ppm (d, 2H); 8,60 ppm (s, 1H); 11,90 ppm (s, 1H)**

Typical procedure for the ε-caprolactam synthesis:
[BMIMSO₃H]HSO₄ (1.49g, 4.7 mmol) and cyclohexanone oxime (0.9 mmol; 0.107 g) (ratio 5/1) were charged into a 25 mL round bottom flask and stirred for 4 hours at 80°C under argon atmosphere. The mixture is then cooled to room temperature and dissolved in 1 mL of water. Then ε-caprolactam is extracted by 5x5 mL of diethylether and dried under vacuum.
The white solid is analyzed by GC / MS and NMR
**NMR (BRUKER 300 MHz AMX, CDCl₃):**
   **¹H: 1,7 ppm (m, 6H); 2,4 ppm (t, J = 6Hz, 2H); 3,2 ppm (t, J = 6Hz, 2H); 6,3 ppm (s, 1H). ¹³C: 23,6 ppm; 29,2 ppm; 30,1 ppm; 37,1 ppm; 43,3 ppm.**
**m/z: 113 (100); 85 (60); 67 (14); 55 (85); 41 (43); 30 (43).**

## Claims

1. A Beckmann rearrangement, wherein a cyclic oxime reacts with a Bronsted acidic ionic liquid of formula 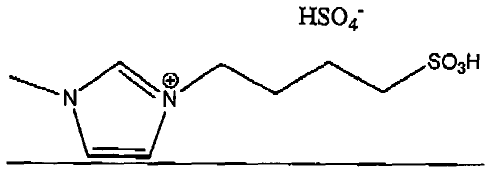 further comprising
a) mechanical stirring of more than 3 hours at more than 70°C wherein the ratio of cyclic oxime to Brønsted acidic ionic liquid is between 1:1 to 1:10 and/or
b) microwave irradiation of more than 30 s, preferably more than 1 min, more preferably between 1 min and 20 min at temperatures of more than 120°C, preferably between 120°C and 250°C and/or
c) ultrasonic treatment of more than 5 s at more than 70 °C at a frequency of between 18 and 800 kHz, preferably 20 to 300 kHz, more preferably 20 to 50 kHz.

2. The method of claim 1 further comprising d) separating the ionic liquid from the lactam.

3. The method of claim 2, further comprising e) reusing the ionic liquid in another reaction.

4. The method of claim 2, further comprising reacting the lactam in a polymerization reaction to form a nylon material.

## Patentansprüche

1. Beckmann-Umlagerung, bei der ein cyclisches Oxim mit einer brönstedsauren ionischen Flüssigkeit der Formel 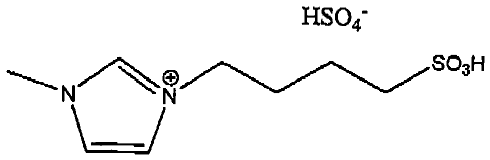 reagiert, ferner umfassend
a) mechanisches Rühren über einen Zeitraum von mehr als 3 Stunden bei mehr als 70°C, wobei das Verhältnis von cyclischem Oxim zu brönstedsaurer ionischer Flüssigkeit zwischen 1:1 und 1:10 liegt, und/oder
b) Mikrowellenbestrahlung über einen Zeitraum von mehr als 30 s, vorzugsweise mehr als 1 min, weiter bevorzugt zwischen 1 min und 20 min, bei Temperaturen von mehr als 120°C, vorzugsweise zwischen 120°C und 250°C, und/oder
c) Ultraschallbehandlung über einen Zeitraum von mehr als 5 s bei mehr als 70°C bei einer Frequenz zwischen 18 und 800 kHz, vorzugsweise 20 bis 300 kHz, weiter bevorzugt 20 bis 50 kHz.

2. Verfahren nach Anspruch 1, ferner umfassend d) Abtrennen der ionischen Flüssigkeit von dem Lactam.

3. Verfahren nach Anspruch 2, ferner umfassend e) Wiederverwenden der ionischen Flüssigkeit in einer anderen Reaktion.

4. Verfahren nach Anspruch 2, ferner umfassend das Umsetzen des Lactams in einer Polymerisationsreaktion zur Bildung eines Polyamidmaterials.

## Revendications

1. Réarrangement de Beckmann, **caractérisé en ce qu'**une oxime cyclique réagit avec un liquide ionique acide de Brønsted de formule 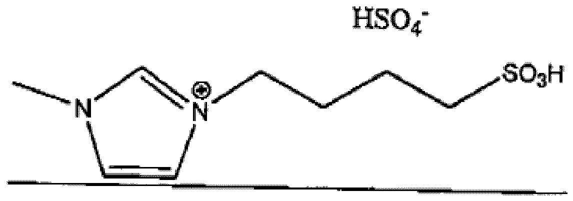 comprenant en outre
a) l'agitation mécanique pendant plus de 3 heures à plus de 70°C, le rapport entre l'oxime cyclique et le liquide ionique acide de Brønsted étant compris entre 1:1 et 1:10 et/ou
b) l'irradiation micro-ondes pendant plus de 30 s, de préférence plus de 1 min, plus préférablement entre 1 min et 20 min à des températures supérieures à 120°C, de préférence comprises entre 120°C et 250°C et/ou
c) le traitement par ultrasons pendant plus de 5 s à plus de 70°C à une fréquence comprise entre 18 et 800 kHz, de préférence entre 20 et 300 kHz, plus préférablement entre 20 et 50 kHz.

2. Méthode selon la revendication 1, comprenant en outre d) la séparation du liquide ionique du lactame.

3. Méthode selon la revendication 2, comprenant en outre e) la réutilisation du liquide ionique dans une autre réaction.

4. Méthode selon la revendication 2, comprenant en outre la réaction du lactame dans une réaction de polymérisation pour former un matériau en nylon.
